# EUROPEAN PATENT APPLICATION

(11) **EP 2 343 103 A1**
(43) Date of publication of application: **13.07.2011**
(21) Application number: 10150499.1
(22) Date of filing: 12.01.2010
(51) Int. Cl.: A61N 7/02, A61N 1/40, A61N 5/10, A61B 17/00, A61B 19/00

(54) **Therapeutic apparatus**

(71) Applicant: Koninklijke Philips Electronics N.V., 5621 BA Eindhoven (NL); Philips Intellectual Property & Standards GmbH, 20099 Hamburg (DE)
(72) Inventor: Katscher, Ulrich, 5600 AE, Eindhoven (NL); Lips, Oliver, 5600 AE, Eindhoven (NL); Findeklee, Christian, 5600 AE, Eindhoven (NL); Leussler, Christoph, 5600 AE, Eindhoven (NL); Nehrke, Kay, 5600 AE, Eindhoven (NL); Wirtz, Daniel, 5600 AE, Eindhoven (NL); Overweg, Johan, 5600 AE, Eindhoven (NL)
(74) Representative: Damen, Daniel Martijn

(57) **Abstract**

The invention provides for a therapeutic apparatus comprising a tissue heating system (302, 480, 482). The therapeutic apparatus further comprises a magnetic resonance imaging system (300) for acquiring magnetic resonance thermometry data (366) from nuclei of a subject (318) located within an imaging volume (330). The therapeutic apparatus further comprises a radiation therapy system (304, 592) for irradiating an irradiation volume (316, 516) of the subject, wherein the irradiation volume is within the imaging volume. The therapeutic apparatus further comprises a controller (354) for controlling the therapeutic apparatus. The controller is adapted for acquiring (100, 210) magnetic resonance thermometry data repeatedly using the magnetic resonance imaging system. The controller is adapted for heating (102, 208) at least the irradiation volume using the tissue heating system. The heating is controlled using the magnetic resonance thermometry data. The controller is adapted for irradiating (104, 208) the irradiation volume.

## Description

### FIELD OF THE INVENTION

The invention relates to a radiation therapy system, in particular a radiation therapy system with magnetic resonance guiding.

### BACKGROUND OF THE INVENTION

In radiation therapy, ionizing radiation is used to selectively destroy regions of tissues within the body of a subject. Radiation therapy is typically used to kill cancerous tumors. Various types of ionizing radiation may be used: charged particles, X-rays, and gamma rays.

A difficulty with radiation therapy is that the ionizing radiation may cause damage to healthy tissue that lies along the path of the ionizing radiation. It is therefore advantageous to minimize the effect of the ionization radiation on health tissue along the path of the ionizing radiation.

United States patent application US2005/0080468 discloses applying ultrasound to a region generate hyperthermia to enhance the effectiveness of radiation therapy.

### SUMMARY OF THE INVENTION

The invention provides for a therapeutic apparatus and a computer program product in the independent claims. Embodiments are given in the dependent claims.

Radiation therapy is one of the most prominent methods of tumor treatment. However, comparing different tumor cells can exhibit significant differences in their sensitivity to radiation, i.e., the probability of radiation induced cell necrosis or apoptosis. The increase of tissue temperature by a few degrees increases the cell's radiation sensitivity, caused by the provoked perfusion increase. This mild form of hyperthermia is below the ablation level, i.e., not the hyperthermia itself damages the tumor cells, but only the combination of hyperthermia and radiation.

Mild hyperthermia may applied in a combined magnetic resonance and radiotherapy system. The hyperthermia might be induced via, e.g., high intensity focused ultrasound or radio-frequency induced hyperthermia. Radio-frequency induced hyperthermia can be performed re-using the magnetic resonance radio-frequency transmission, particularly while using a multi-transmit system.

Tissue heating can be performed via radio-frequency transmission, preferably via an array of transmit antennas. The antenna array could be the same as used for magnetic resonance imaging or a separate array dedicated for hyperthermia. Alternatively, heating could be induced via high-intensity, focused ultrasound.

Temperature can be mapped with magnetic resonance imaging thermometry. Thus, the magnetic resonance system allows real time controlling of the temperature in the target region during heating. Reaching a target temperature may trigger the start of the radiation therapy. During radiation, it is beneficial if the hyperthermia is be maintained to achieve maximum radiation effect. Magnetic resonance thermometry may be used to repeatedly monitor the temperature during the radiation therapy. The use of magnetic resonance thermometry may allow the avoidance of "cold spots" in the irradiation volume as well as "hot spots" with temperatures above ablation level outside the irradiation volume. The irradiation volume is the region of a subject being irradiated by the radiation therapy system.

Various embodiments of the invention may be applied to support radiotherapeutically treated cases of (non-disseminated, non-metastatic) cancer, particularly those with limited radiation sensitivity caused by insufficient perfusion (e.g., cervical cancer, renal cell cancer, melanoma, sarcoma, etc).

The invention provides for a therapeutic apparatus. The therapeutic apparatus comprises a tissue heating system. A tissue heating system as used herein is any apparatus which is adapted for heating a volume or region of a subject. The therapeutic apparatus further comprises a magnetic resonance imaging system for acquiring magnetic resonance thermometry data from nuclei of a subject located within an imaging volume. The magnetic resonance imaging system may also be adapted for acquiring magnetic resonance image data.

Magnetic resonance image data is defined herein as being the recorded measurements of radio frequency signals emitted by atomic spins by the antenna of a magnetic resonance imaging system during a magnetic resonance imaging scan. A magnetic resonance image is defined herein as being the reconstructed two or three dimensional visualization of anatomic data contained within the magnetic resonance imaging data. This visualization can be performed using a computer or a controller.

Magnetic resonance thermometry data is defined herein as being the recorded measurements of radio frequency signals emitted by atomic spins by the antenna of a magnetic resonance imaging system during a magnetic resonance imaging scan which contains information which may be used for magnetic resonance thermometry. Magnetic resonance thermometry functions by measuring changes in temperature sensitive parameters. Examples of parameters that may be measured during magnetic resonance thermometry are: the proton resonance frequency shift, the diffusion coefficient, or changes in the T1 and/or T2 relaxation time may be used to measure the temperature using magnetic resonance. The proton resonance frequency shift is temperature dependent, because the magnetic field that individual protons, hydrogen atoms, experience depends upon the surrounding molecular structure. An increase in temperature decreases molecular screening due to the temperature affecting the hydrogen bonds. This leads to a temperature dependence of the proton resonant frequency.

The apparatus further comprises a radiation therapy system for an irradiation volume of the subject. The irradiation volume is within the imaging volume. The radiation therapy system as used herein is an apparatus adapted for irradiating a volume/region of a subject using ionizing radiation. Ionizing radiation as used herein is either subatomic particles or electromagnetic waves that are energetic enough to break chemical bonds or detach electrons from atoms and molecules. The detachment of an electron from an atom or molecule ionizes them.

The therapeutic apparatus further comprises a controller for controlling the therapeutic apparatus. A controller as used herein is any device adapted for executing machine executable instructions or a program and is adapted for sending control signals to other components or apparatus. Examples of a controller may be, but are not limited to: a microprocessor, a computing device, and a microcontroller. It is also understood that herein a reference to "a controller" may also refer to more than one controller or a collection of different types of controllers. That is to say that the functionality of the controller does not need to be in a single device. There may be multiple controllers, and the controllers may also be integrated into other elements of the therapeutic apparatus.

The controller is adapted for acquiring magnetic resonance thermometry data repeatedly using the magnetic resonance imaging system. The controller is adapted for heating at least the irradiation volume using the tissue heating system. The heating is controlled using the magnetic resonance thermometry data. In some embodiments the magnetic resonance thermometry data is converted into a magnetic resonance temperature map. A magnetic resonance temperature map is a chart or model which contains temperature data about the subject as a function of position within the subject. A magnetic resonance temperature map may be superimposed on a magnetic resonance image or other image which shows anatomical information so that the temperature of various anatomical regions may be displayed. The controller is further adapted for irradiating the irradiation volume using the radiation therapy system. This embodiment is particularly advantageous because the magnetic resonance thermometry data is used to control the heating of the irradiation volume. The heating of tissue makes it more susceptible or more easily damaged by ionizing radiation. Controlling the heating of tissue using the tissue heating system with the magnetic resonance thermometry data allows more accurate heating of tissue and reduces the likelihood of damaging healthy tissue.

In another embodiment the tissue heating system is for heating a heating volume of a subject. The heating volume is within the imaging volume. Because the heating volume is within the imaging volume magnetic resonance thermometry data can be acquired for the heating volume. The irradiation volume is within the heating volume. The controller is a computing device. The computing device comprises a processor. The computing device comprises a computer readable storage medium containing instructions for execution by the processor.

A computing device as used herein refers to any device comprising a processor. A processor is an electronic component which is able to execute a program or machine executable instruction. References to the computing device comprising "a processor" should be interpreted as possibly more than one processor. The term computing device should also be interpreted to possibly refer to a collection or network of computing devices each comprising a processor. Many programs have their instructions performed by multiple processors that may be within the same computing device or which may even distributed across multiple computing device.

A computer-readable storage medium as used herein is any storage medium which may store instructions which are executable by a processor of a computing device. In some embodiments, a computer-readable storage medium may also be able to store data which is able to be accessed by the processor of the computing device. An example of a computer-readable storage medium include, but are not limited to: a floppy disk, a magnetic hard disk drive, a solid state hard disk, flash memory, a USB thumb drive, Random Access Memory (RAM) memory, Read Only Memory (ROM) memory, an optical disk, a magnetooptical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network.

Execution of the instructions causes the at least one processor to perform a method comprising the steps of receiving a treatment plan. A treatment plan as used herein is a detailed plan which comprises operation of the tissue heating system and radiation therapy system for performing a treatment. The treatment plan may comprise anatomical data. The treatment plan may comprise detailed instructions for the operation of the tissue heating system and the radiation therapy system or it may simply contain a plan for heating various regions of tissue with the tissue heating system and a plan for irradiating various volumes of tissue with the radiation therapy system. The heating volume may be larger than the irradiation volume. The treatment plan may contain a plan for heating a heating volume and then a plan for moving the irradiation volume within the heating volume as a function of time. The method further comprises the step of acquiring magnetic resonance image data using the magnetic resonance imaging system.

The method further comprises the step of registering the magnetic resonance image data to the treatment plan. The step of registering the magnetic resonance image data to the treatment plan may comprise constructing a magnetic resonance image using the magnetic resonance image data. The registration of the magnetic resonance image data may comprise the construction of a magnetic resonance image from the magnetic resonance image data. The registration of the magnetic resonance image data to the treatment plan may be achieved by identifying anatomical landmarks within the magnetic resonance image data or magnetic resonance image. The anatomical landmarks may be identified using specialized algorithms and/or rules for particular anatomical regions. The diaphragm may be identified using an edge detection algorithm. For three dimensional magnetic resonance image data a shape constrained deformable model or models may be used to identify anatomical landmarks.

The method further comprises the step of generating a control plan using the registration of the magnetic resonance imaging data to the treatment plan. The control plan comprises machine executable instructions for controlling the magnetic resonance imaging system and for controlling the radiation therapy system. The method further comprises the step of executing the control plan in order to heat the heating volume using the tissue heating system and irradiate the irradiation volume using the irradiation system. Heating the heating volume and irradiation of the irradiation volume may be performed at the same time or they may be performed sequentially. The method further comprises the step of acquiring magnetic resonance thermometry data repeatedly during execution of the control plan using the magnetic resonance imaging system. The method further comprises the step of generating a magnetic resonance temperature map repeatedly during execution of the control plan using the magnetic resonance thermometry data. The method further comprises the step of modifying the control plan repeatedly during execution of the control plan using the magnetic resonance temperature map. This embodiment is particularly beneficial. A treatment plan was received and then registered using magnetic resonance imaging data. The registration of the magnetic resonance imaging data is used to generate a control plan which is then modified repeatedly during execution of the control plan. Use of magnetic resonance thermometry data to repeatedly modify the control plan during its execution allows more accurate control of the heating of tissue within the subject. Radiation damage to tissue may be dependent upon the temperature of the tissue. By controlling the temperature of the tissue during irradiation more accurately the risk of damaging tissue which was not intended to be damaged using the radiation therapy system is reduced.

In another embodiment the irradiation volume is irradiated only when the heated volume is above a first predetermined temperature. The irradiation volume is irradiated only when the region of the subject within the imaging volume and not within the heated volume is below a second predetermined temperature. This embodiment is advantageous because only the heated volume is above a first predetermined temperature. A temperature differential between the first predetermined temperature and the second predetermined temperature can be maintained so as to reduce the likelihood of ionizing radiation damaging tissue within the imaging volume and is not within the heated volume. Raising the temperature of tissue by 5 degrees Celsius may increase the sensitivity of the tissue to radiation. In some embodiments the difference between the first predetermined temperature and the second predetermined temperature may be greater than 4 degrees Celsius. In some embodiments the difference between the first predetermined temperature and the second predetermined temperature may be greater than 5 degrees Celsius.

The method further comprises updating the magnetic resonance image data repeatedly during execution of the control plan. The method further comprises modifying the control plan repeatedly during execution of the control plan using the updated magnetic resonance image data to compensate for motion of the subject. The step of modifying the control plan repeatedly using the updated magnetic resonance image data may comprise the step of constructing a magnetic resonance image using the magnetic resonance image data. The step of updating the magnetic resonance image data may be to acquire magnetic resonance image data for the determination of various anatomical landmarks. Alternatively updating the magnetic resonance image data may be done using the navigator technique. In this technique a limited area of the subject is imaged with magnetic resonance imaging. For instance a region encompassing the edge of the diaphragm may be measured. The anatomical location of other regions of the subject may be inferred from the navigator. As a subject breathes the anatomy of the subject internally deforms in a predictable manner. By tracking the location of the diaphragm with the navigator the motion of the subject can be compensated for during execution of the control plan. This embodiment is beneficial because motion of the subject whether internal or external can be compensated for.

In another embodiment the tissue heating system is a high-intensity focused ultrasound system. A high-intensity focused ultrasound system as used herein is an ultrasound system which is used for heating tissue within a subject and concentrates ultrasound to a region or volume. This focusing may be to a concentrated point or small volume for the purpose of heating tissue or disrupting it mechanically using a process such as cavitation. High-intensity focused ultrasound may also be less focused and may be intended for heating a larger volume.

In another embodiment the tissue heating system is a radio-frequency hyperthermia system. A radio-frequency hyperthermia system uses a radio-frequency antenna or coil and a radio-frequency power supply to heat tissue.

In another embodiment the radiation therapy system is a photon radiation therapy system. A photon radiation therapy system as used herein is a radiation therapy system which uses or generates ionizing radiation using photons. This embodiment is advantageous because high energy photons are easily generated and are not affected by the magnetic field of the magnetic resonance imaging system.

In another embodiment the photon radiation therapy system is any one of the following: a linear accelerator gamma radiation therapy system, an X-ray radiation therapy system and a radioisotope gamma radiation therapy system. A linear accelerator gamma radiation therapy system is a radiation therapy system which uses a linear accelerator to produce gamma radiation. An X-ray radiation therapy system is a radiation therapy system which generates X-rays. A radioisotope gamma radiation therapy system is a radiation therapy system which generates gamma radiation using samples of a radioisotope. The radioisotope gamma radiation therapy system may be a so called gamma knife.

In another embodiment the photon radiation therapy system is a stereotactic radiosurgery radiation therapy system. A stereotactic radiosurgery radiation therapy system uses multiple beams of radiation which all concentrate on a focal point. The purpose of using multiple beams is to reduce the effect on tissue which is not targeted. The radiation is spread over a larger area of the subject and then is concentrated in the irradiation volume.

In another embodiment the radiation therapy system is a charged particle therapy system. A charged particle therapy system is a radiation therapy system which uses accelerated charged particles. If a beam of charged particles is aimed at a subject the charged particles interact with matter in a subject primarily through the Coulomb force. The cross-section for Coulomb collisions increases as the relative velocity of two particles decrease. As a charged particle beam travels through a subject it loses its energy more and more rapidly. The effect of this is that the majority of energy of the particle beam is deposited near the end of the beam path. There is therefore a large peak of energy deposited at the end of the beam path which is called the Bragg peak. This embodiment is advantageous because the majority of energy from the ionizing radiation or the particle beam in this case is deposited in the irradiation volume. The effect on intermediate tissue is minimized.

In another embodiment the charged particle therapy system uses any one of the following: protons, carbon nuclei or other atomic nuclei.

In another embodiment the therapeutic apparatus further comprises a cooling system adapted for cooling the subject. This embodiment is advantageous because as the temperature of a region of tissue increases the likelihood of cells within the tissue being damaged by ionizing radiation increases. If a region of tissue is cooled then the possibility of damage by ionizing radiation decreases. It is therefore advantageous to heat tissue in a heating volume and to cool other tissue which is adjacent to the heating volume and/or tissue through which the ionizing radiation from the radiation therapy system passes.

In another embodiment the cooling system comprises an air chiller adapted for directing chilled air towards the subject. For instance the air chiller could be used to direct air towards a surface region of the subject through which ionizing radiation passes. This would reduce the likelihood of damaging tissue along the path of ionizing radiation which is not within the irradiation volume.

In another embodiment the cooling system comprises a liquid chiller for supplying chilled liquid. This embodiment is advantageous because chilled liquid may be very efficient at cooling a region of the subject.

In another embodiment the cooling system comprises an attachment for supplying the chilled liquid to a saturation bag and/or an implement adapted for insertion into an orifice of the subject. This embodiment is advantageous because a saturation bag or such an implement may be used to chill tissue near the irradiation volume and/or along the path that the ionizing radiation from the radiation therapy system passes through. A saturation bag as used herein is a fluid filled bag which is adapted to be placed on the surface of a subject. The saturation bag may be filled with a material which has the same magnetic susceptibility as fat tissue does. A fat saturation bag may be used to increase the efficiency of a fat saturation pulse sequence for a magnetic resonance imaging system. The addition of the saturation or water bag simulates a more homogeneous volume of tissue which improves the quality of the magnetic resonance image. Also typically such a bag of water is placed between the antenna and the surface of the subject when performing radio-frequency hyperthermia. The reason for this is that when the water bag is there it helps to minimize the magnetic field and maximize the electric field in the region which is to be heated. The term saturation bag as used herein refers to a saturation bag for optimizing fat saturation in magnetic resonance imaging and also for a bag which is used to maximize the electric field in the heating volume when performing radio-frequency hyperthermia. An example of an implement adapted for insertion into an orifice of the subject may be an implement adapted to be inserted into the urethra for chilling tissue around the prostate during the treatment of prostate cancer.

In another embodiment the magnetic resonance imaging system comprises a magnetic field gradient coil adapted for spatial encoding the spins of nuclei within the imaging volume. The radiation therapy system is adapted for producing a radiation beam for irradiating the irradiation volume. The magnetic field gradient coil is a split coil with a split. The radiation therapy system is adapted for aiming the radiation beam through the split. The split may be a physical split where there is no connection or it may be a region of the magnetic field gradient coil where there is no metallic conductor. There may be a metallic connection between the portions of the split coil on either side of the split in a region through which ionizing radiation from the radiation therapy system is never aimed. The magnetic field gradient coil may be embedded in a plastic or resin. As long as the conductor or metallic portion of the magnetic field gradient coil does not intersect the path of the ionizing radiation from the radiation therapy system it will have negligible effect upon the energy or intensity of the ionizing radiation.

In another aspect the invention provides for a computer program product comprising machine executable instructions for execution by a control system of the therapeutic apparatus. The controller is adapted for controlling the therapeutic apparatus. The therapeutic apparatus comprises a tissue heating system. The therapeutic apparatus further comprises a magnetic resonance imaging system for acquiring magnetic resonance imaging data from nuclei of a subject location within an imaging volume. The therapeutic apparatus further comprises a radiation therapy system for irradiating an irradiation volume of the subject. The irradiation volume is within the imaging volume. The machine executable instructions cause the controller to perform a method comprising the step of acquiring magnetic resonance thermometry data repeatedly using the magnetic resonance imaging system. The method further comprises the step of heating at least the irradiation volume using the tissue heating system. The heating is controlled using the magnetic resonance thermometry data. The method further comprises the step of irradiating the radiation volume using the radiation therapy system. The advantages of this have been previously discussed.

### BRIEF DESCRIPTION OF THE DRAWINGS

In the following preferred embodiments of the invention will be described, by way of example only, and with reference to the drawings in which:
Fig. 1 shows a block diagram which illustrates an embodiment of a method according to the invention;
Fig. 2 shows a block diagram which illustrates a further embodiment of a method according to the invention;
Fig. 3 shows a functional diagram of a therapeutic apparatus according to an embodiment of the invention;
Fig. 4 shows a functional diagram of a therapeutic apparatus according to a further embodiment of the invention; and
Fig. 5 shows a functional diagram of a therapeutic apparatus according to a further embodiment of the invention.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

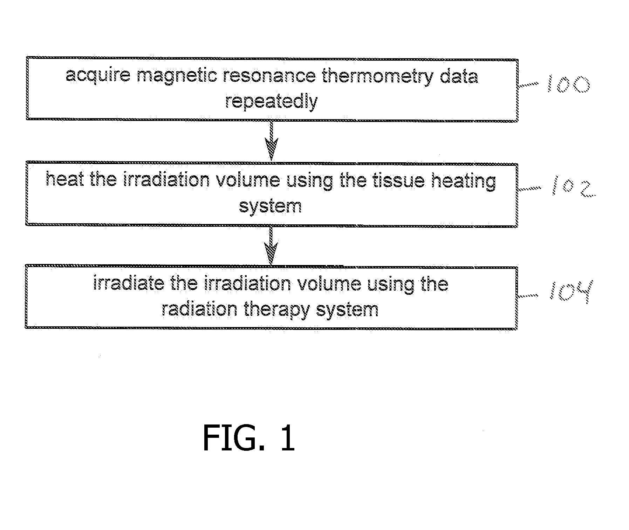

Fig. 1 shows an embodiment of a method according to the invention. In step 100 magnetic resonance thermometry data is acquired repeatedly. In step 102 the irradiation volume is heated using the tissue heating system. In step 104 the irradiation volume is irradiated using the radiation therapy system.

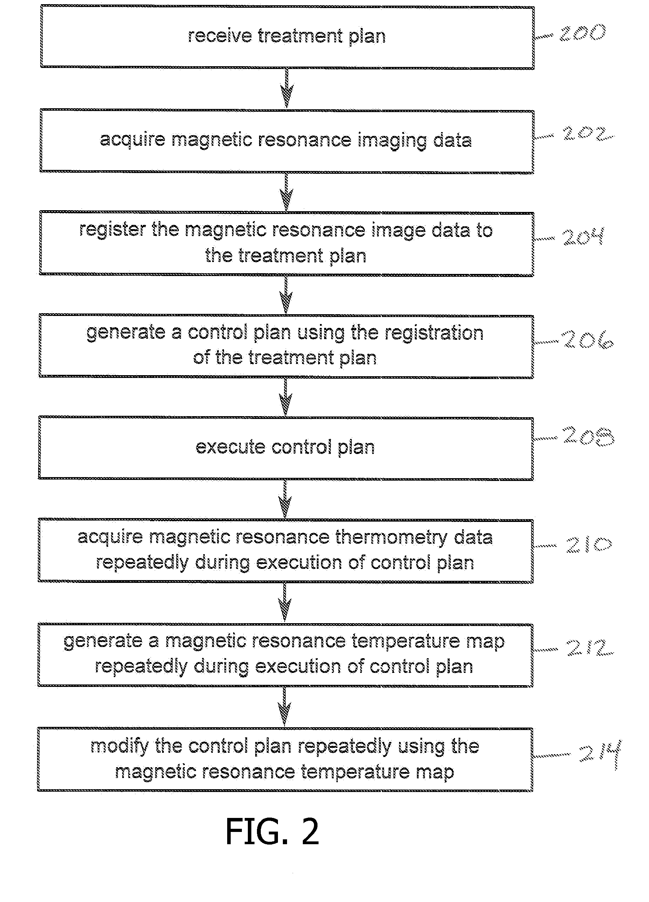

Fig. 2 shows a block diagram which illustrates a further embodiment of a method according to the invention. In step 200 a treatment plan is received. In step 202 magnetic resonance imaging data is acquired. In step 204 the magnetic resonance image data is registered to the treatment plan. The registration of the magnetic resonance image data to the treatment plan establishes a link between the geometry of the magnetic resonance image data to the geometry of the treatment plan. In step 206 a control plan is generated using the registration of the treatment plan. As was explained earlier the control plan contains detailed instructions for the operation of the tissue heating system and the radiation therapy system. In step 208 the control plan is executed. During the execution of the control plan the irradiation volume is heated using the tissue heating system and the irradiation volume is irradiated using the radiation therapy system. In step 210 magnetic resonance thermometry data is repeatedly acquired during execution of the control plan. In step 212 a magnetic resonance temperature map is generated repeatedly during execution of the control plan. The repeatedly acquired magnetic resonance thermometry data is used to generate the magnetic resonance temperature maps. In step 214 the control plan is modified repeatedly using the magnetic resonance temperature map.

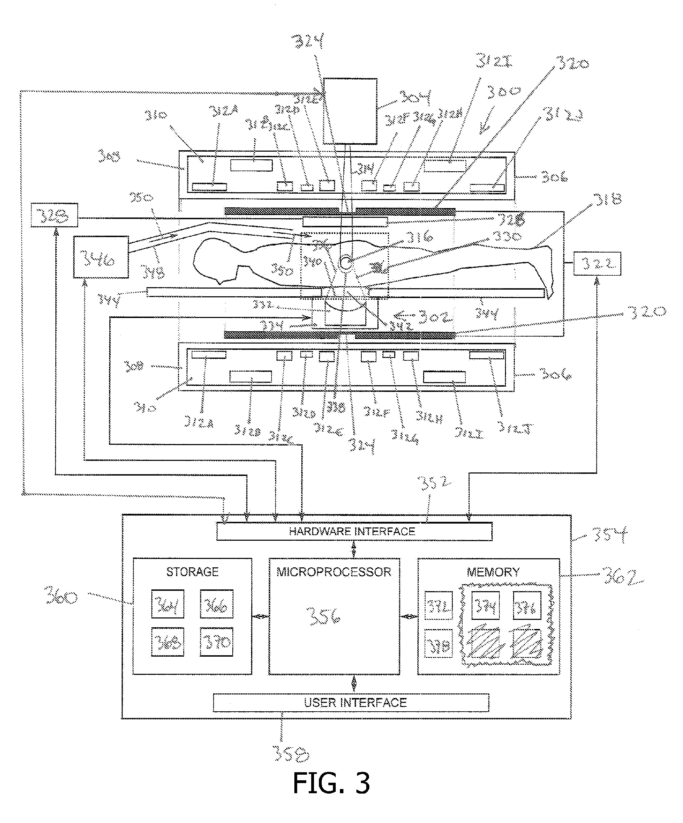

Fig. 3 shows a cross-sectional functional diagram of a therapeutic apparatus according to an embodiment of the invention. The therapeutic apparatus shown in this diagram comprises a magnetic resonance imaging system 300, a high-intensity focused ultrasound system 302 and a photon radiation therapy system 304. The photon radiation therapy system 304 may generate high energy photons using an X-ray source, a linear accelerator or radioisotopes. The photon radiation therapy system 304 may be adapted to rotate about the axis of the magnet 306 of the magnetic resonance imaging system 300. Rotating the photon radiation therapy system 304 about the axis of the magnet allows the radiation to enter a subject 318 from different angles. The magnetic resonance imaging system 300 has a magnet 306. The magnet 306 shown in this embodiment is a cylindrical magnet with superconducting coils. There is in this magnet 306 a vacuum isolation 308 to insulate the magnet from the ambient temperature. Inside of the vacuum isolation 308 is a cryostat 310 filled with liquid helium.

Coils 312a-312j are superconducting coils located within the cryostat 310. This is a cylindrical magnet 306 and the superconducting coils 312a-312j are ring-shaped. For example the superconducting coil 312a in the top section of the magnet 306 is the same coil labeled 312a in the bottom section of the magnet 306. In this Fig. is shown an ionizing radiation beam 314 being emitted from the photon radiation therapy system 304. The ionizing radiation beam 314 passes through or to an irradiation volume 316 within a subject 318. The subject 318 is located within the bore of the magnet 306. The body of the subject 318 is roughly aligned with the axis or z-axis of the magnet 306.

The ionizing radiation beam is able to pass through the magnet 306. The magnet 306 is designed such that the attenuation of the radiation beam by the magnet is minimized. This may be accomplished by moving superconducting coils and other magnet components, such as the gradient coils, out of the path of the radiation beam. The relatively thin walls of the cryostat do not attenuate the radiation beam significantly.

The beam passes between coils 312e and 312f. Also within the bore of the magnet 306 is a magnetic field gradient coil 320. The magnetic field gradient coil 320 is connected to a magnetic field gradient coil power supply 322. When current passes through the magnetic field gradient coil 320 which is supplied by the magnetic field gradient coil power supply 322 the magnetic field gradient coil 320 is able to create magnetic field gradients which can be used to spatially encode atomic spins located within an imaging volume 330 of the magnetic resonance imaging system 300. The magnetic field gradient coil 320 has a split 324.

The magnetic field gradient coil 320 may be two separate gradient coils or the split may simply be a region where there is no or a reduced amount of conducting material. For instance magnet field gradient coils 320 are typically produced in a resin or plastic. They can be designed so that there is no metal within the split region 324 which would interfere with the passing of the ionizing radiation beam 314. The magnet shown in this embodiment 306 is a single coil. In other embodiments a so called open coil could also be used. An open coil has a structure similar to that of a Helmholtz coil. There are two disc-shaped magnets on top of one another and the imaging volume 330 is in between the two disc-shaped magnets. The magnet 306 could also be constructed as two adjacent cylindrical magnets. Also within the bore of the magnet 306 is a radio-frequency transceiver coil 326. The radio-frequency transceiver coil is connected to a radio-frequency transceiver 328. The radio-frequency transceiver coil 326 is used to send radio signals which are able to manipulate the orientation of spins of atoms within the imaging volume 330.

The transceiver coil 326 is also able to receive radio signals emitted by the spins of atomic nuclei within the imaging volume 330. The radio-frequency transceiver coil 326 could be implemented as separate transmitter and receiver coils. Similarly the radio-frequency transceiver could be a discrete transceiver and receiver. The imaging volume 330 is shown as being adjacent to the radio-frequency transceiver coil 326. The imaging volume 330 is the region for which the magnetic resonance imaging system 300 is able to acquire magnetic resonance image data and/or magnetic resonance thermometry data. The high-intensity focused ultrasound system 302 in this embodiment is mounted beneath a subject support 344. The subject support 344 is shown within the bore of the magnet 306. The subject 318 is shown as resting upon the subject support 344. The high-intensity focused ultrasound system 302 has an ultrasound transducer 332 located within a fluid-filled chamber 334. The fluid within the fluid-filled chamber 334 is adapted for transmitting ultrasound. Degassed water may be used for the fluid. The lines 336 indicate the path of the ultrasound from the ultrasonic transducer 332. The path of the ultrasound 336 is focused into a heating volume 338. Ultrasound from the ultrasonic transducer 332 may be focused such that the entire region of the heating volume 338 is heated by the ultrasound transducer 332. Alternatively ultrasound from the ultrasonic transducer 332 may be more highly focused. In this case the ultrasound transducer 332 only heats a portion of the heating volume 338 at any given time. Not shown in this diagram is an electrical power supply for providing electrical power for driving the ultrasonic transducer 332. Also not shown is a mechanical apparatus for physically moving the location of the ultrasonic transducer 332. There is an ultrasonic window 340 which seals the fluid-filled chamber 334. The ultrasonic window 340 is adapted for the transmission of ultrasound. A polyester film such as biaxially-oriented polyethylene terephthalate (boPET) is typically used for the window. There is a gap within the subject support 344 for accommodating a gel pad 342. The gel pad 342 forms an ultrasonic contact between the subject 318 and the ultrasonic window 340.

Also shown in this embodiment is an optional air chiller 346. The air chiller 346 is connected to a tube 348. The air chiller 346 blows chilled air through the tube 348. The arrows labeled 350 indicate the direction of flow of chilled air. The chilled air may be blown towards the subject 318 such that the region of the subject 318 through which the ionizing radiation beam 314 passes through is chilled. This chilling reduces damage to the subject 318 in regions outside of the irradiation volume 316.

The ionizing radiation beam 314 is shown as passing through the magnetic field gradient coil 320 and also the radio-frequency transceiver coil. The radio-frequency transceiver coil may be designed such that the ionizing radiation beam 314 is able to pass through it or a design may be used where a split radio-frequency transceiver coil 326 is used. The high-intensity focused ultrasound system 302, the photon radiation therapy system 304, the magnetic field gradient coil power supply 322, the radio-frequency transceiver 328 and the air chiller 346 are all shown as being connected to a hardware interface 352 of a computing device 354. The hardware interface 352 may be a single hardware interface or it may be a collection of hardware interfaces. The hardware interface 352 allows a processor or microprocessor 356 of the computing device 354 to send control signals to the devices connected to the hardware interface 352. It also allows the reception of data from devices connected to the hardware interface 352. The microprocessor 356 is also connected to a user interface 358. The user interface may be adapted for displaying data or it may also be adapted for receiving input from an operator. The microprocessor 356 is also connected to computer storage 360. Computer storage is an example of a computer-readable storage medium. Computer storage is any non-volatile computer-readable storage medium. Examples of computer storage include, but are not limited to: a hard disk drive, a USB thumb drive, a floppy drive, a smart card, a DVD, a CD-ROM, and a solid state hard drive. In some embodiments computer storage may also be computer memory or vice versa.

The microprocessor 356 is also connected to computer memory 362. Computer memory is an example of a computer-readable storage medium. Computer memory is any memory which is directly accessible to a processor. Examples of computer memory include, but are not limited to: RAM memory, registers, and register files. Within the computer storage 360 is stored a treatment plan 364, magnetic resonance thermometry data 366, magnetic resonance image data 368 and a control plan 370. Within the computer memory 362 is a control module 372. The control module controls executable instructions for controlling the operation of the therapeutic apparatus and its various components. Also located within the computer memory 362 is a registration module 374. The registration module 374 contains code for registering magnetic resonance image data to the treatment plan. Also located within the computer memory 362 is a magnetic resonance reconstruction module 376. The magnetic resonance reconstruction module contains code for rating a magnetic resonance thermometry map from magnetic resonance thermometry data. Also located within the computer memory 362 is a control plan modification module 378. The control plan modification module 378 contains code for modifying the control plan using the magnetic resonance image data 368 and/or the magnetic resonance thermometry data 366. In some embodiments the magnetic resonance reconstruction module may also construct a magnetic resonance image from magnetic resonance image data. The computer storage 360 and the computer memory 362 are both examples of computer-readable storage mediums.

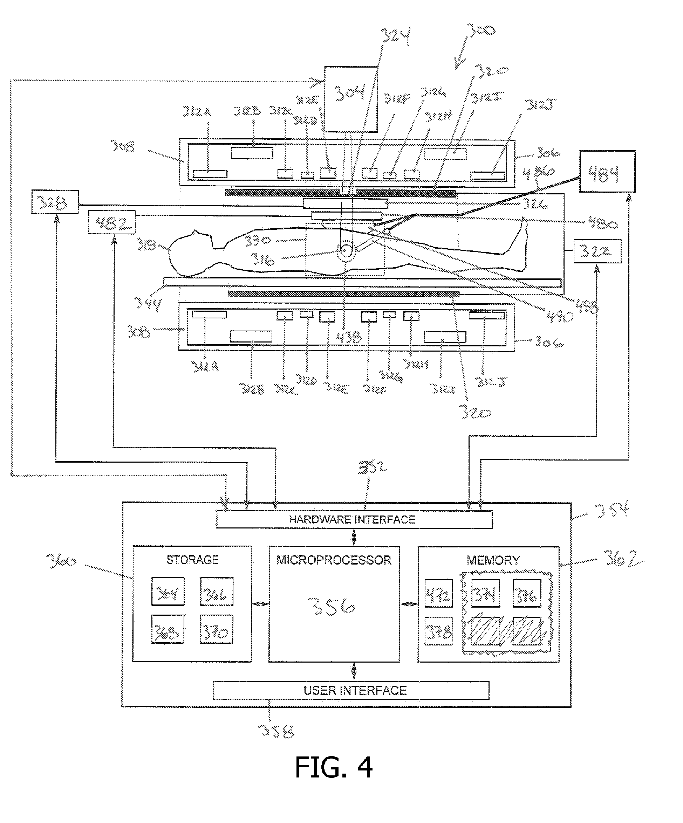

Fig. 4 shows an alternative embodiment of a therapeutic apparatus according to the invention. The embodiment shown in Fig. 4 is similar to the embodiment shown in Fig. 3 with the exception that the high-intensity focused ultrasound system 302 of Fig. 3 has instead been replaced with a radio-frequency hyperthermia system. The radio-frequency hyperthermia system consists of a radio-frequency heating coil 480 and a radio-frequency heating coil power supply 482. Radio-frequency power from the radio-frequency heating coil power supply 482 is used by the radio-frequency heating coil 480 to generate heat within the heating volume 438 of the subject 318. The radio-frequency heating coil power supply 482 is shown as being connected to the hardware interface 352 of the computing device 354. The computer memory 362 contains a control module 472 adapted for controlling the therapeutic apparatus.

Also shown in this figure is an optional fluid chiller 484. The fluid chiller 484 is able to chill fluid. Connected to the fluid chiller 484 is a tube 486 which is connected to the fluid chiller 484. The tube 486 in this embodiment is adapted for forcing the chilled fluid into a saturation bag 488 and an implement 490. The tube 486 is also adapted for returning fluid from the saturation bag 488 and the implement 490. The fluid chiller 484 could be operated with the saturation bag 488 and the implement 490 alone. Both the saturation bag 488 and the implement 490 do not need to be used at the same time. The saturation bag 488 is shown as being between the radio-frequency heating coil 480 and the subject 318. The implement 490 is inserted into an orifice of the subject 318. In this case the implement 490 is inserted into the urethra and is used for chilling the prostate during the treatment of prostate cancer.

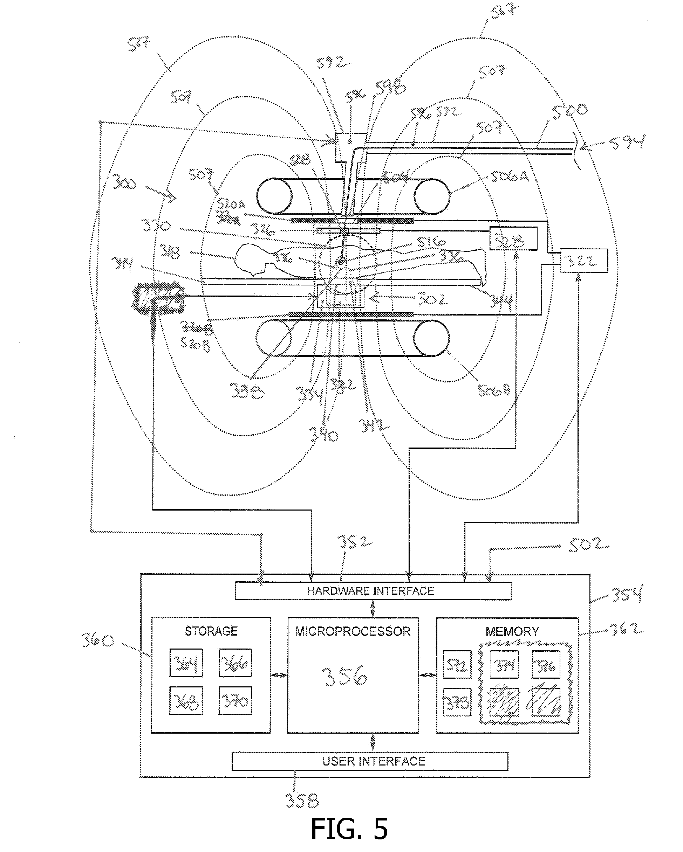

Fig. 5 shows a cross-sectional functional diagram of a further embodiment of the therapeutic apparatus according to an embodiment of the invention. In this embodiment magnetic resonance imaging is combined with high-intensity focused ultrasound and charged particle radiation therapy. The magnet 506a, 506b of the magnetic resonance imaging system is a so called open magnet. The magnet comprises two individual magnets. There is a top magnet 506a and a bottom magnet 506b. Not shown in this figure but there is typically a pedestal which connects the top magnet 506a and the bottom magnet 506b electrically and also the cryogenic system of the two portions 506a, 506b of the magnet.

This arrangement of the magnet creates magnetic field lines similar to what is generated by a Helmholtz coil. The dashed lines 507 show representative magnetic field lines. The imaging volume 330 is in the center between the top magnet 506a and the bottom magnet 506b. The magnetic field gradient coil is divided into a top magnetic field gradient coil 520a and a bottom magnetic field gradient coil 520b. The hardware interface 352 is shown as being connected to the control system 502 of a particle accelerator. The computer memory 362 contains a control module 572 for controlling the operation of the therapeutic apparatus. In addition the control module 572 also contains code for generating commands for controlling the particle accelerator. There is a beam optics 592 which has a connection 594 to a particle accelerator. Within the beam optics 592 is a vacuum 596. The vacuum is within the beam optics so that the charged particle beam 500 is not attenuated within the beam optics 592. The charged particle beam 500 exits the beam optics 592 through a window 598. The window may be constructed of any material which is capable of sealing the vacuum 596 with minimal attenuation of the charged particle beam 500. The window is typically a thin metal plate or foil. The charged particle beam 500 goes through a path 504 through the top magnetic field gradient coil 520a and through a path 508 through the radio-frequency transceiver coil 326. An advantage of using the so called open magnet design is that the path of the particle beam 500 is more closely aligned with the field lines than in a cylindrical magnet. As can be seen in this diagram there is a low angle between the field lines and the charged particle beam 500. The low angle minimizes the deflection of the charged particle beam within the subject 318. The beam optics may contain magnets and/or charged plates for deflecting or adjusting the path of the charged particles in order to direct the path of the charged particles to an irradiation zone 516.

While the invention has been illustrated and described in detail in the drawings and foregoing description, such illustration and description are to be considered illustrative or exemplary and not restrictive; the invention is not limited to the disclosed embodiments.

For example, it is possible to operate the invention in an embodiment wherein additional spatial shaping of the hyperthermia region is performed invasively (by, e.g., magnetic nanoparticle fluids, thermoseeds, or interstitial antennas), or magnetic resonance imaging is performed not only for thermometry but additionally for on-line therapy control (i.e., via monitoring physiologic and / or geometric parameters of the tumor), or the apparatus as described is used to destroy not cancerous but other types of faulty tissue..

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS:

- 300: magnetic resonance imaging system
- 302: high intensity focused ultrasound system
- 304: photon radiation therapy system
- 306: magnet
- 308: vacuum isolation
- 310: cryostat
- 312A - 312 J: superconducting coil
- 314: ionizing radiation beam
- 316: irradiation vo lume
- 318: subject
- 320: magnetic field gradient coil
- 322: magnetic field gradient coil power supply
- 324: split
- 326: radio-frequency transceiver coil
- 328: radio-frequency transceiver
- 330: imaging volume
- 332: ultrasonic transducer
- 334: fluid filled chamber
- 336: path of ultrasound
- 338: heating volume
- 340: ultrasonic window
- 342: gel pad
- 344: subject support
- 346: air chiller
- 348: tube
- 350: direction of chilled air
- 352: hardware interface
- 354: computing device
- 356: processor
- 358: user interface
- 360: computer storage
- 362: computer memory
- 364: treatment plan
- 366: magnetic resonance thermometry data
- 368: magnetic resonance image data
- 370: control plan
- 372: control module
- 374: registration module
- 376: magnetic resonance reconstruction module
- 378: control plan modification module
- 438: heating volume
- 472: control module
- 480: radio-frequency heating coil
- 482: radio-frequency heating coil power supply
- 484: fluid chiller
- 486: tube
- 488: saturation bag
- 490: implement
- 500: charged particle beam
- 502: connection to controller of particle accelerator
- 504: path through top magnetic field gradient coil
- 506A: top magnet
- 506B: bottom magnet
- 507: magnetic field line
- 508: path through radio-frequency transceiver coil
- 516: irradiation zone
- 520A: top magnetic field gradient coil
- 520B: bottom magnetic field gradient coil
- 572: control module
- 592: beam optics
- 594: connection to particle accelerator
- 596: vacuum
- 598: window

## Claims

1. A therapeutic apparatus comprising:
- a tissue heating system (302, 480, 482);
- a magnetic resonance imaging system (300) for acquiring magnetic resonance thermometry data (366) from nuclei of a subject (318) located within an imaging volume (330),
- a radiation therapy system (304, 592) for irradiating an irradiation volume (316, 516) of the subject, wherein the irradiation volume is within the imaging volume; and
- a controller (354) for controlling the therapeutic apparatus, wherein the controller is adapted for acquiring (100, 210) magnetic resonance thermometry data repeatedly using the magnetic resonance imaging system, wherein the controller is adapted for heating (102, 208) at least the irradiation volume using the tissue heating system, wherein the heating is controlled using the magnetic resonance thermometry data, wherein the controller is adapted for irradiating (104, 208) the irradiation volume using the radiation therapy system.

2. The therapeutic apparatus of Claim 1, wherein the tissue heating system is for heating a heating volume (338, 438) of the subject; wherein the heating volume is within the imaging volume; wherein the irradiation volume is within the heating volume; wherein the controller is a computing device (354); wherein the computing device comprises a processor (356), wherein the computing device comprises a computer-readable storage medium (360, 362) containing instructions for execution by the processor, wherein execution of the instructions causes the processor to perform a method comprising the steps of:
- receiving (200) a treatment plan (364);
- acquiring (202) magnetic resonance image data using the magnetic resonance imaging system;
- registering (204) the magnetic resonance image data to the treatment plan;
- generating (206) a control plan (370) using the registration of the magnetic resonance imaging data to the treatment plan, wherein the control plan comprises machine executable instructions for controlling the magnetic resonance imaging system and for controlling the radiation therapy system;
- executing (208) the control plan in order to heat the heating volume using the tissue heating system and irradiate the irradiation volume using the irradiation system;
- acquiring (210) magnetic resonance thermometry data (366) repeatedly during execution of the control plan using the magnetic resonance imaging system;
- generating (212) a magnetic resonance temperature map repeatedly during execution of the control plan using the magnetic resonance thermometry data; and
- modifying (214) the control plan repeatedly during execution of the control plan using the magnetic resonance temperature map.

3. The therapeutic apparatus of any one of Claims 2, wherein the irradiation volume is irradiated only when the heated volume is above a first predetermined temperature; and wherein the irradiation volume is irradiated only when the region of the subject within the imaging volume and not within the heated volume is below a second predetermined temperature.

4. The therapeutic apparatus of any one of Claim 2 or 3, wherein the method further comprises:
- updating the magnetic resonance image data repeatedly during execution of the control plan; and
- modifying the control plan repeatedly during execution of the control plan using the updated magnetic resonance image data to compensate for motion of the subject.

5. The therapeutic apparatus of any one of the preceding Claims, wherein the tissue heating system is a high intensity focused ultrasound system (302).

6. The therapeutic apparatus of Claims 1 through 4, wherein the tissue heating system is a radio-frequency hyperthermia system (480, 482).

7. The therapeutic apparatus of any one of the preceding Claims, wherein the radiation therapy system is a photon radiation therapy system (304).

8. The therapeutic apparatus of Claim 7, wherein the photon radiation therapy system is any one of the following: a linear accelerator gamma radiation therapy system, an X-ray radiation therapy system, and a radioisotope gamma radiation therapy system.

9. The therapeutic apparatus of any one of Claims 1 through 6, wherein the radiation therapy system is a charged particle therapy system (592).

10. The therapeutic apparatus of any one of the preceding Claims, wherein the therapeutic apparatus further comprises a cooling system (346, 484) adapted for cooling the subject.

11. The therapeutic apparatus of Claim 10, wherein the cooling system comprises an air chiller (346) adapted for directing chilled air towards the subject.

12. The therapeutic apparatus of Claim 10 or 11, wherein the cooling system comprises a liquid chiller (484) for supplying chilled liquid.

13. The therapeutic apparatus of Claim 12, wherein the cooling system comprises an attachment (486) for supplying the chilled liquid to a saturation bag (488) and/or an implement (490) adapted for insertion into an orifice of the subject.

14. The therapeutic apparatus of any one of the preceding Claims, wherein the magnetic resonance imaging system comprises a magnetic field gradient coil (320) adapted for spatially encoding the spins of the nuclei within the imaging volume, wherein the radiation therapy system is adapted for producing a radiation beam for irradiating the irradiation volume, wherein the magnetic field gradient coil is a split coil with a split (324), wherein the radiation therapy system is adapted for aiming the radiation beam through the split (324).

15. computer program product comprising machine executable instructions for execution by a control system (354) of a therapeutic apparatus; wherein the controller is adapted for controlling the therapeutic apparatus; wherein the therapeutic apparatus comprises a tissue heating system (302, 480, 482); wherein the therapeutic apparatus further comprises a magnetic resonance imaging system (300) for acquiring magnetic resonance thermometry data (366) from nuclei of a subject located within an imaging volume (330); wherein the therapeutic apparatus further comprises a radiation therapy system (304, 592) for irradiating an irradiation volume of the subject; wherein the irradiation volume is within the imaging volume; wherein the machine executable instructions cause the controller to perform a method comprising the steps of:
- acquiring (100, 210) magnetic resonance thermometry data repeatedly using the magnetic resonance imaging system;
- heating (102, 208) at least the irradiation volume using the tissue heating system, wherein the heating is controlled using the magnetic resonance thermometry data; and
- irradiating (104, 208) the irradiation volume using the radiation therapy system.
